(19) Europäisches Patentamt
European Patent Office
Office européen des brevets

(11) Publication number: **0 065 400**
**B1**

(12) # EUROPEAN PATENT SPECIFICATION

(45) Date of publication of patent specification: **05.03.86**

(21) Application number: **82302366.8**

(22) Date of filing: **10.05.82**

(51) Int. Cl.[4]: **C 01 B 33/28,** C 01 B 33/20, C 01 B 35/12, C 01 G 17/00, C 01 G 28/02, C 01 G 30/02, C 01 G 31/00, C 01 G 37/14, C 01 G 39/00, C 01 G 45/12, C 01 G 49/00

(54) **Zeolites.**

(30) Priority: **20.05.81 GB 8115410**
**22.02.82 GB 8205109**

(43) Date of publication of application:
**24.11.82 Bulletin 82/47**

(45) Publication of the grant of the patent:
**05.03.86 Bulletin 86/10**

(84) Designated Contracting States:
**BE DE FR GB IT NL**

(56) References cited:
**EP-A-0 015 132**
**FR-A-2 387 908**

(73) Proprietor: **IMPERIAL CHEMICAL INDUSTRIES PLC**
**Imperial Chemical House Millbank**
**London SW1P 3JF (GB)**

(72) Inventor: **Hogan, Philip John**
**25 Cheltenham Crescent**
**Runcorn Cheshire (GB)**
Inventor: **Stewart, Allan**
**18 Springbourne**
**Frodsham Cheshire (GB)**
Inventor: **Whittam, Thomas Vincent**
**30 Wilton Drive**
**Darlington Cleveland (GB)**

(74) Representative: **Martin, David Lincoln et al**
**Imperial Chemical Industries PLC Legal Department: Patents PO Box 6 Bessemer Road**
**Welwyn Garden City Herts, AL7 1HD (GB)**

**EP 0 065 400 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a novel zeolite material hereinafter referred to as zeolite Nu-10, to a method of making it and to processes using it as a catalyst.

According to the present invention, we provide a synthetic zeolite material, designated zeolite Nu-10, having a molar composition expressed by the following formula:

$$0.5 \text{ to } 1.5\ R_2O{:}Y_2O_3{:}\ \text{at least } 20\ XO_2{:}0 \text{ to } 4000\ H_2O$$

wherein R is a monovalent cation or 1/n of a cation of valency n, X is silicon and/or germanium, Y is one or more of aluminium, iron, chromium, vanadium, molybdenum, arsenic, antimony, manganese, gallium or boron, and $H_2O$ is water of hydration additional to water notionally present when R is H, and having an X-ray pattern substantially as set out in Table 1 (as determined by standard technique using copper K $\alpha$ radiation).

Zeolite Nu-10 is characterised in part by its interplanar spacings as shown in Table 1. However, to characterise a zeolite fully, X-ray data alone is inadequate, and sorption and catalytic properties must also be taken into account. A further feature of zeolite Nu-10, its unique infra red spectra also helps in its characterisation.

TABLE 1
X-ray data of zeolite Nu-10

| d(A) | I |
|---|---|
| 10.95±0.25 | m→s |
| 8.80±0.14 | w→m |
| 6.99±0.14 | w→m |
| 5.41±0.10 | w |
| 4.57±0.09 | w |
| 4.38±0.08 | vs |
| 3.69±0.07 | vs |
| 3.63±0.07 | vs |
| 3.48±0.06 | m→s |
| 3.36±0.06 | w |
| 3.31±0.05 | w |
| 2.78±0.05 | w |
| 2.53±0.04 | m |
| 2.44±0.04 | w |
| 2.37±0.03 | w |
| 1.88±0.02 | w |

vs=60 to 100
s=40 to 60
m=20 to 40
w= 0 to 20

Within the above definition, particular mention may be made of materials having a molar composition expressed by the formula

$$0.5 \text{ to } 1.5\ R_2O{:}Y_2O_3{:}\text{at least } 60{\times}O_2{:}0 \text{ to } 200\ H_2O$$

The $XO_2/Y_2O_3$ ratio in Nu-10 is typically in the range 20 to 5000. The sodium/organo forms of Nu-10 appear to be most readily formed when this ratio is in the range 80 to 120. On the other hand, with potassium or rubidium as inorganic cation, Nu-10 is most readily formed with a $XO_2/Y_2O_3$ ratio in the range 40 to 1000.

This definition includes both freshly prepared Nu-10 ("freshly prepared" means the product of synthesis, and washing, with optional drying, as hereinafter described) and also forms of it resulting from dehydration, and/or calcination, and/or ion exchange. In freshly prepared Nu-10, R may include an alkali metal cation, especially sodium, potassium, rubidium or caesium. Ammonium and hydrogen may also be present. Usually, or when prepared from nitrogen compounds, Nu-10 includes nitrogen-containing organic cations or bases as described below or cationic degradation products thereof, or precursors thereof. These nitrogen containing cations or bases are hereinafter referred to as Q.

The freshly prepared Nu-10 may also contain nitrogen-containing compounds well in excess of the 1.5 moles set out in the aforesaid definition of the composition of Nu-10 typically in the range 0.1 to 120 moles per mole of $Y_2O_3$. Since Nu-10 is a zeolite, the nitrogen containing base must be physically trapped within the crystal lattice. It can be removed by thermal treatment and/or oxidative degradation or by displacement by suitable small molecules. This physically trapped basic material does not constitute part of the composition for the purposes of the definition. Thus Nu-10 as made typically has the following molar composition:

$$0.5 \text{ to } 1.5\ M_2O : 0.1 \text{ to } 120\ Q : Y_2O_3 : 20 \text{ to } 5000\ XO_2 : 0 \text{ to } 2000\ H_2O$$

wherein M is an alkali metal and/or ammonium and can include hydrogen.

Among the ion-exchanged forms of zeolite Nu-10 the ammonium ($NH_4^+$) is of importance since it can be readily converted to the hydrogen form by calcination. The hydrogen form can also be prepared directly by exchange with an acid. The hydrogen-form and forms containing metals introduced by ion exchange are described further below.

The sorption results given in Table 2 were obtained on the hydrogen Nu-10 of Example 12 which had been calcined at only 450°C, and still contained carbonaceous residues (0.9% carbon by weight). When this material was further calcined for 17 hours at 550°C in moist air the carbon reduced to 0.2%$^w$. While the equilibrium sorption capacities were not significantly affected and were substantially as in Table 2, the rates of sorption, except for m-xylene, were very much higher. Thus 90% equilibrium was achieved within 10 minutes and there was no significant increase in sorption of n-hexane, pyridine or p-xylene after 2 hours. On the other hand, in the HNu-10 calcined at 550°C, the m-xylene still only gave surface coverage and failed to enter the zeolite lattice.

These results and the results given in Table 2 suggest that zeolite Nu-10 has 10 ring ports which are a tight fit for pyridine and p-xylene at 25°C. Both diffuse in slowly after an initial modest uptake, suggesting a substantial diffusion barrier exists at 25°C for these molecules i.e. at about 5.85A. The sorption of m-xylene corresponds only to surface coverage on the zeolite Nu-10 powder. This slow uptake of n-exhane shows the channels in Nu-10 present length barriers to molecules as well as imposing restraints due to cross sectional areas. Thus Nu-10 can be expected to show unique sorptive and catalytic properties not hitherto seen in highly siliceous zeolites. Zeolite Nu-10 should prove useful in separating aromatic compounds especially isomers e.g. xylenes and ethylbenzene, and in separating molecules not only on cross sectional area differences, but also on length differences. It is likely that substantial changes in separation processes and in shape-selective catalytic processes can be achieved by simply changing the temperature of operation and thus modifying the effects of energy barriers to diffusion.

TABLE 2
Sorption on hydrogen Nu-10 (from Example 12) at 25°C

| Sorbate | Water | n-hexane | Pyridine | p-xylene | m-xylene |
|---|---|---|---|---|---|
| Relative pressure p/po | 0.3 | 0.33 | 0.3 | 0.5 | 0.5 |
| Kinetic diameter σA* | 2.7 | 4.3 | 5.8 | 5.85 | 6.0 |
| Sorption % w/w<br>10 min | 3.8 | 1.8 | 2.4 | 1.7 | 0.5 |
| 1 hour | 4.0 | 2.2 | 2.6 | 1.9 | |
| 2 hour | 4.0 | 2.4 | 2.7 | 2.0 | |
| 24 hour | | 4.5 | 5.8 | 3.0 | |
| 68 hour | 4.3 | 4.8 | 6.9 | 6.3 | 0.8 |

*Lennard Jones kinetic diameter (D. W. Breck, "Zeolite Molecular Sieves" Wiley Interscience (1974), p. 636).

TABLE 3
Voidage available in HNu-10 at 25°C

| Sorbate | Kinetic diameter σA | Wt sorbed g/100 g | Apparent voidage filled at equilibrium cc/100 g |
|---|---|---|---|
| water | 2.7 | 4.3 | 4.3 |
| n-hexane | 4.3 | 4.8 | 7.2 |
| pyridine | 5.8 | 6.9 | 7.1 |
| p-xylene | 5.85 | 6.3 | 7.2 |
| m-xylene | 6.0 | 0.8 | 0.9 |

Table 3 gives comparative data on apparent voidage filled by different sorbates assuming that all sorbates are adsorbed as liquids, with typical liquid densities at 25°C.

Zeolite Nu-10 is further characterised by its infra-red spectrum (shown in the attached drawing). In common with other zeolites, zeolite Nu-10 has two main IR absorption regions, viz. the stretch ν of the Si—O situated near to 1100 $cm^{-1}$ and the deformation δ of the Si—O situated near to 500 $cm^{-1}$. Referring to the absorptions near to 1100 $cm^{-1}$, zeolite Nu-10 has three distinct absorptions at 1209 $cm^{-1}$ medium, 1117 $cm^{-1}$ strong and 1081 $cm^{-1}$ strong. By comparison, the absorptions near to 1100 $cm^{-1}$ for ZSM-5 are 1228 $cm^{-1}$ (medium) and 1095 $cm^{-1}$ (strong). Thus there is a significant difference both in position and number of absorptions of zeolite Nu-10 as compared with the absorptions of ZSM-5.

The deformations δ near to 500 $cm^{-1}$ of Nu-10 again shows three characteristic, clearly defined absorptions at 637 $cm^{-1}$ (weak), 547 $cm^{-1}$ (weak) and 463 $cm^{-1}$ (medium strong). By comparison, ZSM-5 has a doublet absorption at 545 $cm^{-1}$ (medium) and 455 $cm^{-1}$ (medium). The number of absorptions, their positions and relative intensities found in the infrared spectrum of zeolite Nu-10 are sufficient to identify Nu-10 and to distinguish it from ZSM-5.

Zeolite Nu-10 may be prepared by reacting an aqueous mixture containing at least one oxide $XO_2$, at least one oxide $Y_2O_3$ and at least one polyalkylene polyamine having the formula:

$$\begin{array}{c} R^1 \\ N \\ H \end{array} - \left[ (CH_2)_x - NH \right]_y - (CH_2)_x - \begin{array}{c} R^2 \\ N \\ H \end{array}$$

where x is an integer from 2 to 6 and y is an integer from 0 to 10, provided that when y is 0, x is 2 to 5, each of $R^1$ and $R^2$, independently, represents hydrogen or a $C_1$—$C_6$ alkyl group and wherein the reaction mixture has the molar composition

$XO_2/Y_2O_3$=25 to 5000, preferably 40 to 1000, most preferred 80 to 500
$M^1OH/XO_2=10^{-8}$ to 1.0, preferably $10^{-6}$ to 0.25, most preferred $10^{-4}$ to 0.15
$H_2O/XO_2$=10 to 200, preferably 15 to 60, most preferred 30 to 50
$Q/XO_2$=0.05 to 4, preferably 0.1 to 1.0, most preferred 0.2 to 0.5
$M^2Z/XO_2$=0 to 4.0, preferably 0 to 1.0, most preferred 0 to 0.6

Zeolite Nu-10 can be prepared in the temperature range 5 to 250°C. The preferred range is 90 to 180°C and crystallization is carried out until substantially pure crystalline Nu-10 is obtained. Agitated reactions are preferred.

X is silicon or germanium, Y is aluminium, iron, chromium, vanadium, molybdenum, arsenic, antimony, mangenese, gallium or boron, $M^1$ is an alkali metal or ammonium or hydrogen, $M^2$ is an alkali metal or ammonium or hydrogen and can be the same as $M^1$ and Q is the aforesaid polyalkylene polyamine, amine degradation product thereof or a precursor thereof, or a related compound. $Z^-$ is a strong acid radical present as a salt of $M^2$ and may be added as a free acid to reduce the free $M^1OH$ level to a desired value.

$M^2Z$ addition, while not essential, can accelerate crystallization of zeolite Nu-10 and can also affect the size of crystals obtained. It should be noted that addition of $M^2Z$ can promote the formation of α-quartz and α-crystobalite, this can usually be avoided by reducing reaction times and/or temperature to minimise the chances of overrun. If $M^2Z$ remains intercalated within the zeolite Nu-10 framework, then it can have substantial effects on the sorption and catalytic properties. $M^1$ and/or Q can be present as hydroxides or salts of inorganic or organic acids provided the $M^1OH/XO_2$ requirement is fulfilled.

The preferred polyalkylene polyamines are triethylene tetramine and tetraethylene pentamine.

The preferred alkali metal $M^1$ is sodium and/or potassium $M^2$ is preferably the same as $M^1$ or is hydrogen. The preferred oxide $XO_2$ is silica ($SiO_2$) and the preferred oxide $Y_2O_3$ is alumina ($Al_2O_3$).

The silica source can be any of those commonly considered for use in synthesising zeolites, for example powdered solid silica, silicic acid, colloidal silica or dissolved silica. Among the powdered silicas usable are precipitated silicas, especially those made by precipitation from an alkali metal silicate solution, such as the type known as "KS 300" made by AKZO, and similar products aerosil silicas, fume silicas and silica gels suitably in grades for use in reinforcing pigments for rubber or silicone rubber. Colloidal silicas of various particle sizes may be used, for example 10—15 or 40—50 microns, as sold under the Registered Trade Marks "Ludox", "Nalcoag" and "Syton". The usable dissolved silicas include commercially available waterglass silicates containing 0.5 to 0.6, especially 2.0 to 4.0 mols of $SiO_2$ per mol of alkali metal oxide, "active" alkali metal silicates as defined in UK Patent 1193254, and silicates made by dissolving silica in alkali metal hydroxide or quaternary ammonium hydroxide or a mixture thereof.

The alumina source is most conveniently sodium aluminate, but can be or can include aluminium, an aluminium salt for example the chloride, nitrate or sulphate, an aluminium alkoxide or alumina itself, which should preferably be in a hydrated or hydratable form such as colloidal alumina, pseudoboehmite, boehmite, gamma alumina or the alpha or beta trihydrate.

The reaction mixture is reacted usually under autogenous pressure, optionally with added gas, e.g. nitrogen at a temperature between 50 and 250°C until crystals of Nu-10 form, which can be from 1 hour to many months depending on the reactant composition and the operating temperature. Agitation is optional, but is preferable since it reduces the reaction time. If agitation is inadequate, or the reaction is carried out under quiescent conditions, then there is a distinct probability of producing zeolite Nu-10 contaminated by ZSM5 family zeolites, such as zeolite Nu-4 of our copending UK Patent Application No. 8115407.

In order to prepare the best possible adsorbents and catalysts from zeolite Nu-10, it is essential that the levels of ZSM5 family zeolites are negligible, because ZSM5 family zeolites give rise to undesirable side effects as a result of their inferior shape-size-selectivity-performance as compared with the unique and highly desirable properties of zeolite Nu-10 for certain applications, for example including but not limited to the separation of aromatic isomers, toluene methylation, and dehydration of alcohols.

The synthesis of zeolite Nu-10 in substantially pure form can be guaranteed by strict adherence to the narrow range conditions. For reaction mixture compositions in the wider ranges, substantially pure Nu-10 can still be obtained, especially where the more important narrow range criteria such as $XO_2/Y_2O_3$, $M^1OH/XO_2$ and temperature/time are met. Time/temperature becomes more critical in the wider range and overrun to α-quartz and α-cristobalite becomes more likely. This tendency can be reduced by using potassium or rubidium as the alkali cations. To those skilled in the art it will be apparent, from examples given, that even in the wider reaction mixture ranges, many combinations will still give Nu-10 as the major component, and that with care it will be possible to prepare pure Nu-10.

If mixtures of zeolite Nu-10 and ZSM5 family zeolites are desired then this can be achieved by deliberately operating at $XO_2/Y_2O_3$ ratios below 70 and $NaOH/XO_2$ greater than 0.06.

At the end of the reaction, the solid phase is collected on a filter and washed and is then ready for further steps such as drying, dehyration and ion-exchange.

If the product of the reaction contains alkali metal ions, these have to be at least partly removed in

order to prepare more acid forms of Nu-10 and this can be done by ion exchange with an acid, especially a strong mineral acid such as hydrochloric acid or by way of the ammonium compound, made by ion exchange with a solution of an ammonium salt such as ammonium chloride. Such ion exchange can be carried out by slurrying once or several times with the solution.

In general, the cation(s) of zeolite Nu-10 can be replaced by any cation(s) of metals, and particularly those in Groups IA, IB, IIA, IIB, III (including rare earths) VIII (including noble metals) and by lead, tin and bismuth. (The Periodic Table is as in "Abridgements of Specifications" published by the UK Patent Office). Exchange is carried out using any water soluble salts containing the appropriate cation.

In order to prepare a catalyst, zeolite Nu-10 may be used in association with an inorganic matrix, or with other materials which can be either inert or catalytically active. The matrix may be present simply as a binding agent to hold the small zeolite particles (0.005 to 10 microns) together, or it may be added as a diluent to control the amount of conversion in a process which may otherwise proceed at too high a rate, leading to catalyst fouling as a result of excessive coke formation. Typical inorganic diluents include catalyst support materials such as alumina, silica, kaolinic clays, bentonites, montmorillonites, sepiolite, attapulgite, Fullers earth, synthetic porous materials such as $SiO_2$—$Al_2O_3$, $SiO_2$—$ZrO_2$, $SiO_2$—$ThO_2$, $SiO_2$—BeO, $SiO_2$—$TiO_2$ or any combination of these oxides. An effective way of mixing zeolite Nu-10 with such diluents is to mix appropriate aqueous slurries in a mixing nozzle and then to spray dry the slurry. Other ways of mixing can be used.

If zeolite Nu-10 in any cationic form or as a catalytic composite is exchanged or impregnated with hydrogenation/dehydrogenation components, such as Ni, Co, Pt, Pd, Re, Rh, hydrocracking and reforming catalysts can be made, especially if the $Na_2O$ content is less than 0.1% w/w.

A wide range of hydrocarbon conversion catalysts can be prepared from zeolite Nu-10 by ion exchange or impregnation with cations, or oxides, selected from the following, Cu, Ag, Mg, Ca, Sr, Zn, Cd, B, Al, Sn, Pb, V, P, Sb, Cr, Mo, W, Mn, Re, Fe, Co, Ni and noble metals.

Usually, the Nu-10 catalyst will be in acid form thus stoichiometry is maintained by $H^+$ or $H_3O^+$ as an additional balancing cation, or as sole cation. Such catalysts may find application in the following processes: catalytic cracking, hydrodesulphurization, hydrodenitrification, catalytic dewaxing, alkylation of alkanes or aromatics, dealkylation, disproportionation, isomerisation of alkanes and alkyl benzenes, dehydration reactions, oxidation and polymerisation.

Particular mention may be made of an alkylation process which comprises contacting an alkyl-benzene or a mixture of alkylbenzenes and an alkylating agent under alkylating conditions in the vapour or liquid phase with a catalyst comprising zeolite Nu-10.

The alkylbenzene starting materials include toluene, ortho-, meta- and para-xylenes, ethylbenzene, trimethylbenzene, tetramethylbenzene, and the like, or mixtures thereof. The alkylating process is especially applicable to the use of toluene as the starting material.

Suitable alkylating agents include alkanols, alkyl halides, alkyl ethers, alkyl sulphides and olefins. Preferred methylating agents include methanol, methyl chloride, methyl bromide, methyl carbonate, dimethyl ether and dimethyl sulphide. The use of methanol as the methylating agent is especially preferred.

The molar ratio of the alkylating agent to the alkylbenzene is generally between about 0.005 and about 5, for example between about 0.1 and about 3.

An especially preferred alkylating process comprises the methylation of toluene using methanol as the methylating agent to give a product comprising the xylene isomers, and in particular to a process for the selective production of para-xylene from toluene and methanol, the said para-xylene being obtained in excess of its normal equilibrium concentration (which is about 23—24%) of the xylene isomers with limited production of higher methylbenzenes.

The alkylation process, e.g. methylation, is suitably carried out at a temperature in the range of about 250 to about 750°C, preferably about 350°C, to about 600°C, at a pressure between 1 atmosphere abs and about 60 atmosphere abs and at a WHSV of 1 to about 1500.

Mention may also be made of a process for the production of hydrocarbons which comprises contacting a lower monohydric alcohol or an ether derived therefrom under conversion conditions with a catalyst comprising zeolite Nu-10.

Lower monohydric alcohols which may be used in this process particularly include saturated monohydric alcohols containing up to four carbon atoms in the molecule. Ethers which may be used include the symmetrical and unsymmetrical ethers derived from the aforesaid alcohols by dehydration. If desired, mixtures of alcohols and/or ethers may be used. It is particularly preferred to use methanol and/or dimethyl ether as the starting material(s).

This process is suitably carried out at a temperature in the range 250—700°C and preferably in the range 350—500°C. The pressure is suitably in the range 0.2—50 atm abs, preferably 0.5 to 20 atm abs. The weight hourly space velocity (WHSV) is typically in the range of about 0.5 to 50.

The products of the reaction, which include lower olefins, especially $C_2$ to $C_7$ olefins, particularly propylene and butylenes, and smaller amounts of some aromatic hydrocarbons such as benzene, toluene and xylenes, may be recovered and separated using conventional methods.

The catalyst may be in the form of a fixed bed, fixed fluid bed or it may be of the transport type. The catalyst as used maintains its activity for a substantial period, but can be regenerated by heating.

For example, when the catalyst is used in the form of a fluidised bed, the catalyst may be continuously withdrawn, passed through a regeneration zone and returned to the reaction.

Zeolite Nu-10 may also find applications in pollution control by its ability to remove organic contaminants from aqueous effluents.

The invention is illustrated by the following Examples.

Example 1

Preparation of sodium tetraethylene pentamine (TEPA) Nu-10 and sodium Nu-10

The synthesis mixture had the following molar composition:

$$2.34\ Na_2O,\ 27\ TEPA,\ Al_2O_3,\ 96.6\ SiO_2,\ 3878\ H_2O,\ 54.8\ NaCl$$

38 g TEPA were dispersed in 142 g colloidal silica (Syton X-30). Next, 1.6 g sodium aluminate (1.25 $Na_2O$, $Al_2O_3$, $3H_2O$) dissolved in 10 g water were stirred into the silica suspension followed by 23 g sodium chloride dissolved in 388 g water. The resulting gel was mixed thoroughly for ½ hour to a homogeneous texture, and was then reacted for 3 days at 180°C in a 1 litre stainless steel stirred autoclave under autogenous pressure. The stirrer speed was maintained at 500 rpm. After cooling to about 60°C, the slurry was filtered and washed with 2 litres of distilled water at 60°C and dried overnight at 120°C. The product was sodium TEPA Nu-10 with X-ray diffraction data as shown in Table 4.

Example 2

The product of Example 1 was calcined in moist air for 48 hours at 450°C and its X-ray diffraction pattern was substantially unchanged. Next, it was slurry-exchanged for 1 hour at 25°C with 4 ml of N hydrochloric acid per g of zeolite. The resulting hydrogen Nu-10 was washed with 10 ml of distilled water per g of zeolite, dried overnight at 120°C, and calcined in moist air for 6 hours at 450°C. Its X-ray diffraction data were still substantially as given in Table 4.

Example 3

This Example was the same as Example 1 except that TEPA was replaced by a molar equivalent of triethylene tetramine (TETA). The product after 3 days at 180°C was sodium TETA NU-10, as shown by X-ray data given in Table 5.

TABLE 4

| | | | | | | | | | |
|---|---|---|---|---|---|---|---|---|---|
| dA | 10.9 | 9.94 | 8.76 | 6.97 | 5.373 | 4.575 | 4.353 | 4.095 | |
| 100I/Io | 40 | 11 | 6 | 11 | 9 | 10 | 100 | 26* | |
| | | | | | | | | | |
| dA | 3.663 | 3.619 | 3.466 | 2.988 | 2.818 | 2.522 | 2.493 | 2.439 | 2.368 |
| 100I/Io | 94 | 74 | 31 | 8 | 8* | 26 | 5* | 8 | 6 |

*due at least in part to α-cristobalite

TABLE 5

| | | | | | | | |
|---|---|---|---|---|---|---|---|
| dA | 10.9 | 6.97 | 5.373 | 4.564 | 4.353 | 4.086 | 3.678 |
| 100I/Io | 11 | 13 | 10 | 10 | 100 | 32* | 90 |
| | | | | | | | |
| dA | 3.619 | 3.480 | 2.818 | 2.522 | 2.495 | 2.436 | 2.362 |
| 100I/Io | 72 | 34 | 25 | 22 | 9* | 11 | 10 |

*due at least in part to α-cristobalite

Example 4

This example demonstrates that if high levels of TETA are employed then reaction times must be reduced or substantial contamination by α-cristobalite occurs. The Example was exactly as Example 3 but 94.9 g of TETA were used instead of 27.2 g which corresponds to an increase in the $Q/SiO_2$ ratio of from 0.28

to 0.94. The product after 2 days was Nu-10 with about 5% cristobalite as an impurity, but after 3 days the α-cristobalite level had increased to about 25%.

Example 5

This example illustrates that zeolite Nu-10 can be prepared from mixtures containing very little free alkali e.g. $M^1OH/SiO_2$=0.0099. The molar composition of the mixture was:

18 TEPA, 0.48 $Na_2O$, $Al_2O_3$, 96.3 $SiO_2$, 3600 $H_2O$, 54 NaCl, 1.82 $Na_2SO_4$.

18 g TEPA were dispersed in 152 g colloidal silica. Next 1.5 g sodium aluminate (1.19 $Na_2O$, $Al_2O_3$, 1.29 $H_2O$) dissolved in 10 g water were stirred in, followed by 24.3 g sodium chloride and 1.4 g concentrated sulphuric acid in 458 g water. The reaction was carried out as in Example 1, except that the reaction time was 3½ days. The product was an excellent sample of zeolite Nu-10.

Example 6

This Example illustrates that for some reaction mixture compositions with $SiO_2/Al_2O_3$ ratio greater than about 200, zeolite Nu-10 can be very heavily contaminated. The molar composition of the reaction mixture was:

7.36 $Na_2O$, 281 TETA, $Al_2O_3$, 300 $SiO_2$, 11293 $H_2O$, 166 NaCl.

The mixture was reacted at 180°C for 3 days with stirrer speed 500 rpm and the product contained about 60% α-quartz and approximately equal amounts of zeolite Nu-10, zeolite Nu-4 and α-cristobalite. The products after 1 and 2 days contained only amorphous materials.

Example 7

Sodium tetraethylene pentamine Nu-10 was prepared as follows:

Solution A

142 g Syton ×30 colloidal silica (Monsanto chemicals of molar composition 27 $Na_2O$:2400 $SiO_2$:$Al_2O_3$:19550 $H_2O$)

37.8 g Tetraethylenepentamine (TEPA)—"Technical" grade British Drug Houses).

Solution B

1.6 g Sodium Aluminate ("Technical" grade, British Drug Houses, of molar composition 1.22 $Na_2O$:$Al_2O_3$:1.02 $H_2O$)

10 g Deionised water

Solution C

22.5 g Sodium chloride ("Analar" grade, British Drug Houses)

388 g Deionised water

This reaction mixture had the following molar composition:

2.14 $Na_2O$:84 $SiO_2$:$Al_2O_3$:25 TEPA:48 NaCl:3421 $H_2O$

Solution A was introduced into a one litre 316 stainless steel autoclave, equipped with an air driven magnedrive turbine stirrer (manufactured by Autoclave Engineers). Solution B was added with stirring at ambient temperature. Solution C was then added with stirring until a homogeneous gel was produced. After sealing the autoclave, the reaction mixture was maintained under autogenous pressure with stirring (approximately 1000 rpm) at 180°C for 5 days. At the end of this time, the reaction mixture was cooled to room temperature and the product filtered and washed with deionised water (3 litres) and dried for several hours at 150°C, yield ca. 40 g.

Examination of the crystals by scanning electron microscopy (SEM) showed these to be needles of uniform width of 500 Å and lengths varying between ½ and 1 μ. The product exhibited an X-ray powder diffraction pattern as shown in Table 6 and the following elemental analyses: Carbon 2.84 wt.%, Hydrogen 0.67 wt.%, Nitrogen 1.65 wt.%, Silicon 42.9 wt.%, Aluminium 0.95 wt.%. The product had a $SiO_2/Al_2O_3$ ratio of 87.

TABLE 6
X-ray data of sodium T.E.P.A. Nu-10 prepared as described in Example 7

| Interplanar spacing d (Å) | Relative intensity 100 I/Io |
|---|---|
| 10.77 | 49 |
| 8.66 | 19 |
| 6.91 | 19 |
| 5.40 | 13 |
| 4.53 | 16 |
| 4.33 | 100 |
| 4.07 | 69* |
| 3.66 | 98* |
| 3.62 | 85 |
| 3.46 | 36 |
| 2.51 | 58* |
| 2.43 | 14 |
| 2.37 | 9 |
| 1.87 | 8 |
| 1.60 | 8 |
| 1.43 | 9 |

*due at least in part to α-cristobalite

The hydrogen form of Nu-10 was prepared by calcining 10 g of the above product in air at 450°C for 17 hours, then exchanging at ambient temperature with 0.1 molar hydrochloric acid solution (ca. 300 $cm^3$) for several hours and finally drying at 150°C for 3 hours. This hydrogen Nu-10 had the following elemental analysis. Carbon 0.44 wt.%, Hydrogen 0.31 wt.%, Nitrogen<0.02 wt.%, Silicon 44.4 wt.%, Aluminium 0.94 wt.%. $SiO_2/Al_2O_3$ was 91.

Example 8
The reaction was carried out as in Example 7 with the exception that 1.6 g NALFLOC sodium aluminate (of molar composition 1.23 $Na_2O$:$Al_2O_3$:2.8 $H_2O$) was used at a reaction temperature of 150°C for 3½ days. The reaction mixture had the following molar composition:

$$2.24\ Na_2O:95\ SiO_2:Al_2O_3:25\ TEPA:53\ NaCl:3852\ H_2O$$

The product was shown by S.E.M. to be 1000 to 1500 Å diameter by 2 to 3 μ long needles and exhibited the X-ray powder diffraction of sodium TEPA Nu-10 as shown in Table 7. It was calcined and exchanged as in Example 7. The respective X-ray data of the resulting sodium/hydrogen Nu-10 and the hydrogen Nu-10 are also included in Table 7.

Example 9
The reaction was carried out as in Example 7 with the exception that the reaction was at a temperature of 150°C for 4 days. The product was shown to be 500 to 1000 Å diameter by 1 to 2 μ long needles by S.E.M. and exhibited an X-ray powder diffraction as shown in Table 8. The product was calcined and exchanged as in Example 7.

Example 10

The reaction was carried out as in Example 7 with the exception that the reaction was at a temperature of 120°C for 10 days. The product exhibited an X-ray powder diffraction as shown in Table 9.

Example 11

The reaction was carried out as in Example 7 with the exception that 18.9 g of TEPA was used at a reaction temperature of 105°C for 40 days. The product exhibited an X-ray powder diffraction as shown in Table 10.

Example 12

The molar composition of the synthesis mixture was

$$2.32\ Na_2O,\ 90.6\ TETA,\ Al_2O_3,\ 96.3\ SiO,\ 3862\ H_2O$$

94.5 g TETA were dispersed in 141.5 g colloidal silica, followed by a solution of 1.6 g sodium aluminate in 396.5 g water. The mixture was homogenised for 30 minutes, and then transferred to a 1 litre stainless steel autoclave. The reaction was carried out as in Example 1. The dried product was a very pure sample of sodium TETA Nu-10 zeolite of molar composition:

$$1.08\ Na_2O,\ 2.7\ TETA,\ Al_2O_3,\ 90\ SiO_2,\ 12\ H_2O$$

and consisted of rod shaped crystals with dimensions of 1 µm long by 0.1 µm thick.

X-ray diffraction data is given in Table 11.

A portion of this product was calcined in air at 450°C for 72 hours. The resulting sodium hydrogen Nu-10 had the X-ray data shown in Table 11.

The sample of sodium hydrogen Nu-10 was slurry exchanged for 1 hour at 60°C with 5 ml per g of normal hydrochloric acid. The product was filtered and washed twice with 10 ml/g of distilled water, dried at 120°C and finally calcined for 3 hours at 450°C in air. Its molar composition was:

$$0.07\ Na_2O,\ Al_2O_3,\ 92.1\ SiO_2$$

and it contained 0.9% w/w carbon and had the X-ray diffraction data shown in Table 11.

This hydrogen Nu-10 product consisted of needles 1.0—1.5 µm in length and 0.1 µm in thickness and there was very substantial twinning. The crystal type was C centred orthorhombic with a probable space group CMCM and unit cell a=13.853, b=17.434, c=5.040.

TABLE 7

| Sodium TEPA Nu-10 d(Å) | 100 I/Io | Sodium hydrogen NU-10 d(Å) | 100 I/Io | Hydrogen Nu-10 d(Å) | 100 I/Io |
|---|---|---|---|---|---|
| 10.64 | 36 | 10.77 | 57 | 10.64 | 61 |
| 8.58 | 17 | 8.66 | 23 | 8.58 | 19 |
| 6.91 | 17 | 6.91 | 28 | 6.86 | 24 |
| 5.37 | 12 | 5.40 | 17 | 5.37 | 18 |
| 4.55 | 17 | 4.55 | 17 | 4.53 | 19 |
| 4.35 | 98 | 4.33 | 100 | 4.33 | 99 |
| 3.64 | 100 | 3.66 | 91 | 3.64 | 100 |
| 3.60 | 74 | 3.62 | 66 | 3.59 | 67 |
| 3.45 | 40 | 3.45 | 37 | 3.44 | 41 |
| 3.32 | 10 | 3.28 | 6 | 3.28 | 8 |
| 2.51 | 24 | 2.51 | 22 | 2.51 | 23 |
| 2.43 | 10 | 2.43 | 12 | 2.43 | 12 |
| 2.35 | 7 | 2.36 | 8 | 2.35 | 9 |
| 1.87 | 7 | 1.87 | 9 | 1.86 | 8 |
| 1.59 | 5 | — | — | — | — |

TABLE 8

X-ray data of sodium TEPA Nu-10 prepared as described in Example 9

| Interplanar spacing d(Å) | Relative intensity 100 I/Io |
|---|---|
| 10.91 | 48 |
| 8.75 | 18 |
| 6.91 | 19 |
| 5.40 | 12 |
| 4.55 | 16 |
| 4.35 | 100 |
| 3.66 | 96 |
| 3.62 | 78 |
| 3.46 | 39 |
| 2.51 | 26 |
| 2.43 | 12 |
| 2.36 | 8 |

TABLE 9

X-ray data of sodium T.E.P.A. Nu-10 prepared as described in Example 10

| Interplanar spacing d(Å) | Relative intensity 100 I/Io |
|---|---|
| 10.77 | 44 |
| 8.66 | 16 |
| 6.96 | 17 |
| 5.40 | 14 |
| 4.57 | 14 |
| 4.35 | 94 |
| 3.66 | 100 |
| 3.62 | 79 |
| 3.48 | 38 |
| 2.51 | 14 |
| 2.43 | 11 |
| 2.36 | 10 |

TABLE 10

X-ray data of sodium T.E.P.A. Nu-10 prepared as described in Example 11

| Interplanar spacing d(Å) | Relative intensity 100 I/Io |
|---|---|
| 10.77 | 48 |
| 8.66 | 17 |
| 6.91 | 18 |
| 5.37 | 12 |
| 4.55 | 15 |
| 4.35 | 96 |
| 3.66 | 100 |
| 3.62 | 77 |
| 3.46 | 40 |
| 2.51 | 24 |
| 2.44 | 12 |
| 2.36 | 8 |

TABLE 11
X-ray data for zeolite Nu-10 (Example 12)

| As-made Nu-10 | | Sodium hydrogen Nu-10 | | Hydrogen Nu-10 | |
|---|---|---|---|---|---|
| dA | 100I/Io | dA | 100I/Io | dA | 100I/Io |
| 11.19 | 51 | 10.92 | 78 | 10.98 | 66 |
| 8.93 | 6 | 8.76 | 14 | 8.76 | 11 |
| 6.970 | 16 | 6.971 | 20 | 6.971 | 18 |
| 5.471 | 9 | 5.439 | 13 | 5.422 | 12 |
| 4.611 | 12 | 4.576 | 13 | 4.588 | 12 |
| 4.418 | 97 | 4.375 | 100 | 4.375 | 100 |
| 3.708 | 100 | 3.693 | 92 | 3.693 | 90 |
| 3.655 | 75 | 3.626 | 63 | 3.626 | 65 |
| 3.500 | 38 | 3.467 | 34 | 3.467 | 30 |
| 3.370 | 10 | 3.347 | 8 | 3.339 | 6 |
| 3.320 | 8 | 3.303 | 7 | 3.303 | 5 |
| 2.536 | 20 | 2.526 | 20 | 2.529 | 22 |
| 2.449 | 11 | 2.439 | 9 | 2.439 | 9 |
| 2.380 | 7 | 2.374 | 6 | 2.371 | 7 |
| — | — | 2.067 | 5 | 2.072 | 4 |
| — | — | 2.038 | 5 | 1.907 | 3 |
| 1.884 | 6 | 1.870 | 3 | 1.874 | 7 |

TABLE 12
X-ray data of potassium, sodium TEPA Nu-10 of Example 19

| Interplanar spacing d(Å) | Relative intensity 100 I/Io |
|---|---|
| 10.79 | 59 |
| 8.93 | 4 |
| 8.68 | 9 |
| 7.22 | 3 |
| 6.93 | 12 |
| 5.40 | 10 |
| 5.32 | 7 |
| 4.543 | 7 |
| 4.355 | 61 |
| 3.665 | 87 |
| 3.616 | 100 |
| 3.467 | 45 |
| 3.342 | 8 |
| 3.215 | 2 |
| 2.971 | 3 |
| 2.938 | 4 |
| 2.898 | 4 |
| 2.777 | 4 |
| 2.736 | 5 |
| 2.518 | 14 |
| 2.451 | 9 |
| 2.435 | 12 |
| 2.366 | 8 |
| 2.066 | 4 |
| 2.037 | 5 |
| 2.001 | 4 |
| 1.900 | 5 |
| 1.878 | 8 |

Example 13

The molar composition of the reaction mixture was

$$3.6\ Na_2O,\ 42.6\ TETA,\ Al_2O_3,\ 150\ SiO_2,\ 6000\ H_2O,\ 54\ NaCl.$$

The reaction was carried out as in Example 1. The product contained about 85% zeolite Nu-10 along with 15% α-cristobalite.

Example 14

The molar composition of the reaction mixture was

$$1.21\ Na_2O,\ 26\ TETA,\ Al_2O_3,\ 96.3\ SiO_2,\ 2014\ H_2O,\ 54.8\ NaCl$$

The reaction was carried out as in Example 1. A sample withdrawn after 48 hours was substantially pure zeolite Nu-10, but the product after 72 hours at 180°C was zeolite Nu-10 plus about 40% α-cristobalite.

Example 15

The synthesis mixture had the following molar composition.

$$8.4\ Na_2O,\ 57\ Q,\ Al_2O_3,\ 170\ SiO_2,\ 8500\ H_2O$$

51 g of solid silica (Degussa Aerosil 200) were dispersed in a mixture of 24 g ethylene diamine and 740 g water. Next, 1 g sodium aluminate (1.22 $Na_2O_2$, $Al_2O_3$, 1.9 $H_2O$) and 5.3 g sodium hydroxide were dissolved in 25 g water. Finally, the aluminate solution was stirred into the silica suspension and then reacted for 6 days at 150°C in a stirred stainless steel 1 litre autoclave. The product was Nu-10 having an $SiO_2/Al_2O_3$ of 165.

Example 16

This Example illustrates that symmetrically di-substituted ethylene diamines can be used in Nu-10 synthesis. The synthesis mixture had the following molar composition

$$143\ K_2O,\ 54Q,\ Al_2O_3,\ 170\ SiO_2,\ 8000\ H_2O$$

48 g aerosil 200 were dispersed in a mixture of 29.5 g N,N$^1$-diethyl ethylene diamine and 702 g water. Next 0.75 g Kaiser S.A. alumina were dissolved in 7.5 g potassium hydroxide and 16.5 g water. The aluminate solution was stirred into the silica suspension and reacted for 41 hours at 180°C in a stirred 1 litre stainless steel autoclave. The Nu-10 product had a $SiO_2/Al_2O_3$ of 169.

By contrast, when unsymmetrical N,N-diethyl ethylene diamine was used the reaction yielded a pure sample of zeolite Nu-4 of our copending application Number 8115407.

Example 17

This Example was exactly as Example 1 except that 20.5 g ethylene diamine replaced the TEPA. The reaction was for 5 days at 150°C and the product was Nu-10.

Example 18

The synthesis mixture had the following molar composition.

$$1.89\ Na_2O,\ 20.4Q,\ Al_2O_3,\ 60.5\ SiO_2,\ 2563\ H_2O,\ 32.0\ NaCl,\ 1.69\ H_2SO_4$$

First, 43 g of TEPA and 141 g colloidal silica were mixed. Next, 2.2 g sodium aluminate (1.09 $Na_2O$, $Al_2O_3$, 2.33 $H_2O$) dissolved in 10 g water were stirred in followed by 20 g sodium chloride and 1.8 g concentrated sulphuric acid in 380 g water.

The effective $OH^-/SiO_2$ of this reaction was 0.007. The mix was reacted for 4 days at 180°C and the NU-10 product had a $SiO_2/Al_2O_3$ ratio of 56.

Example 19

The synthesis mixture had the following composition.

$$1.22\ Na_2O,\ 40.8\ K_2O,\ 120\ TEPA,\ Al_2O_3,\ 500\ SiO_2,\ 25{,}000\ H_2O$$

46 g Degussa Aerosil 200 were dispersed in 35 g TEPA and 660 g water. Next, 0.3 g sodium aluminate (1.22 $Na_2O$, $Al_2O_3$, 1.2 $H_2O$) and 7 g potassium hydroxide were dissolved in 31 g water, and then were mixed into the silica suspension. The mixture was reacted for 5 days at 180°C in a stirred stainless steel autoclave. The product was a highly crystalline potassium, sodium TEPA Nu-10 having a $SiO_2/Al_2O_3$=492 and the X-ray data given in Table 12.

Examples 20—21

Example 20, was as Example 19 except that the potassium hydroxide was replaced by 10.7 g rubidium hydroxide.

The product after 7 days at 180°C was rubidium, sodium TEPA Nu-10 of $SiO_2/Al_2O_3$=500. Example 21 was as Example 19 except that the potassium hydroxide was replaced by 18.7 g caesium hydroxide. The product after 5 days at 200°C was caesium, sodium Nu-10 plus about 10% each of α-cristobalite and zeolite ZSM-48.

Example 22

This Example was the same as Example 19 except that 1 g of boric acid was added to the potassium hydroxide, sodium aluminate solution. The product after 8 days at 160°C was Nu-10 of $SiO_2/Al_2O_3$=475 and $SiO_2/B_2O_3$=640. About 10% α-cristobalite was present as an impurity.

Example 23

This Example was as Example 19 except that 1.5 g antimonious oxide was stirred into the final reaction mixture. The product after 8 days at 160°C contained 0.3% w/w $Sb_2O_3$.

Examples 24—25

In these Examples, other additives were mixed into Example 19 type reactions and reaction was for 8 days at 160°C. The additives were dissolved in the water prior to dispersing the solid silica.

In Example 24, the addition was 2 g potassium chrome alum and the dried Nu-10 product had a $SiO_2/Al_2O_3$=600 and a $SiO_2/Cr_2O_3$=1100.

In Example 25, the additive was 0.7 g disodium hydrogen phosphate and the Nu-10 product had a $SiO_2Al_2O_3$=700 and a $SiO_2$/P=830.

Example 26

The synthesis mixture had the following molar composition

$$4.2\ K_2O,\ 17\ TEPA,\ 50\ SiO_2,\ 2500\ H_2O.$$

46.2 g Aerosil 200 were dispersed in a mixture of 49.4 g TEPA and 643 g water. Next, 2.4 g Kaiser S.A. alumina ($Al_2O_3$, $3H_2O$) was dissolved in a solution of 7.2 g potassium hydroxide in 24 g water. The aluminate was stirred into the silica dispersion and the mixture was reacted for 8 days at 180°C. The product was potassium Nu-10 having $SiO_2/Al_2O_3$=47.

Example 27

This example illustrates the use of HNu-10 as a catalyst for toluene methylation. Zeolite HNu-10 was prepared as described in Example 8.

Approximately 2 g of this batch of HNu-10 was compressed, crushed and sieved. 0.35 g of 250—500 μ HNu-10 catalyst particles were loaded into a microreactor in which the zeolite was tested for toluene methylation with methanol as methylating agent. Prior to contact with reactants, the catalyst bed was flushed with nitrogen at 450°C for about 1 hour. A feed consisting of toluene and methanol in a 3:1 molar ratio of the aromatic compound to the alcohol was vaporised and passed over the catalyst at 450°C at a WHSV of about 7.7. The progress of the reaction is outlined by the results in Table 13.

The proportion of p-xylene in the xylenes fraction is clearly considerably in excess of the equilibrium concentration of 24% and the selectivity to xylenes in the methylated products is also high. The trimethylbenzenes produced in this test were predominantly 1,2,4-trimethylbenzene (~90%). This test, with a calcination of the catalyst included, demonstrates that the catalyst can be regenerated to give approximately the same level of activity and selectivity in the conversion of toluene to methylated products and, in particular, p-xylene.

TABLE 13

| Time on stream hrs | Conversion of toluene to methylated product wt.% | Mol % of methylated product Xylenes | Mol % of methylated product Trimethylbenzenes | Wt.% p-xylene in xylenes fraction |
|---|---|---|---|---|
| 1.75 | 16.1 | 91.2 | 8.8 | 63.9 |
| 13.00 | 6.5 | 94.6 | 5.4 | 62.7 |
| 25.00 | Reactant flow ceased; after $N_2$ flush, the catalyst was calcined in the reactor at 530°C for 16 hours with about 60 ml $m^{-1}$ flow of air. | | | |
| 1.35* | 17.2 | 90.6 | 9.4 | 64.0 |

*after recommencement of reactant mixture flow, reactor temp. 450°C

Example 28

This Example illustrates the use of HNu-10 as a catalyst for toluene methylation. Zeolite HNu-10 was prepared as described in Example 9.

Approximately 2 g of this batch of HNu-10 was compressed, crushed and sieved. 0.37 g of 250—500 μ HNu-10 catalyst particles were loaded into a microreactor and tested in a similar fashion to that described in Example 16 except that the WHSV was about 7.4. The progress of the reaction is outlined by the results in Table 14.

It is clear that the proportion of p-xylene in the xylenes fraction is considerably in excess of the equilibrium concentration of 24% and the selectivity to xylenes in the methylated products is also high. The trimethylbenzenes produced in the test were predominantly 1,2,4-trimethylbenzene (~90%).

The results of this test are thought to be characteristic of a relatively pure specimen of HNu-10.

Example 29

This Example illustrates the use of HNu-10 as a catalyst for toluene methylation. The HNu-10 used in this test was prepared using triethylenetetramine as described in Example 12.

Approximately 2 g of this HNu-10 was compressed, crushed and sieved. 0.34 g of 250—500 μ catalyst particles were loaded into a microreactor and heated to 450°C in a stream of nitrogen. A feedstock consisting of toluene and methanol in a 3:1 molar ratio was reacted with the catalyst in the microreactor in a similar way to that described in Example 16, except that the flow rate was equivalent to a WHSV of about 9.25. The progress of this test, carried out at approximately atmospheric pressure, is outlined by the results in Table 15.

It is clear that the proportion of p-xylene in the xylenes fraction is considerably in excess of the equilibrium concentration of 24% and the selectivity to xylenes in the methylated products is also high.

TABLE 14

| Time on stream hrs | Conversion of toluene to methylated product wt.% | Mol % of methylated product | | Wt.% p-xylene in xylenes fraction |
|---|---|---|---|---|
| | | Xylenes | Trimethylbenzenes | |
| 3.85 | 28.2 | 87.1 | 12.9 | 59.2 |
| 4.85 | 25.1 | 88.2 | 11.8 | 62.8 |
| 5.75 | Reactant mixture flow stopped; the catalyst was purged for 16 hours with nitrogen at 450°C. | | | |
| 3.50* | 23.4 | 89.9 | 10.1 | 61.7 |

*After recommencement of reactant mixture flow to catalyst.

TABLE 15

| Time on stream hrs | Toluene conversion wt.% | Mol % methylated products | | Mol % p-xylene in xylenes fraction |
|---|---|---|---|---|
| | | Xylenes | Trimethylbenzenes | |
| 3.3 | 19.4 | 92.0 | 8.0 | 58.9 |
| 6.1 | 16.2 | 92.1 | 7.9 | 57.6 |

Example 30

This Example is used to illustrate the effectiveness of HNu-10 as a catalyst for toluene disproportionation. The HNu-10 used in this test was that prepared in Example 8.

Approximately 2 g of this HNu-10 was compressed, crushed and sieved. 0.38 g of 250—500 μ HNu-10 catalyst particles were loaded into a microreactor and heated to 530°C in a stream of nitrogen. The sample was maintained at this temperature in a stream of nitrogen for a further hour before toluene was passed to the catalyst. Toluene was vaporised and fed to the catalyst at a rate equivalent to a WHSV of 11.1. The progress of this test, carried out at approximately atmospheric pressure and with neat toluene feed, is outlined by the results given in Table 16.

TABLE 16

| Time on stream hrs | Toluene conversion wt.% | Mol % p-xylene in xylenes fraction |
|---|---|---|
| 1.0 | 5.4 | 31.9 |
| 3.1 | 0.7 | 51.2 |

It will be evident that the amount of p-xylene present in the xylenes fraction is greater than that found in an equilibrium mixture (~24% p-xylene) but the catalyst shows relatively low activity for toluene disproportionation.

Example 31

A sample of sodium TEPA Nu-10 prepared as described in Example 8 was calcined at 550°C for 16 hours in air. The calcined material was then slurry-exchanged with 50 ml N/l $NH_4$ Cl per g of zeolite for 16 hours at 100°C. The sample was filtered, washed with distilled water and then dried at 110°C. The $NH_4$-NU-10 was then calcined at 550°C for 16 hours in air to produce H-Nu-10. Chemical analysis of the resulting material indicated that it had the following molar composition ignoring hydrogen:

$$0.02Na_2O \,.\, Al_2O_3 \,.\, 98SiO_2$$

In a continuous flow reactor, about 1 g of the catalyst, particle size 710—1000 μ, was tested for methanol conversion. The catalyst was activated at 450°C under helium for 1 hour. The temperature was reduced to 400°C then methanol in helium was passed over the catalyst. The conditions and analysis of the $C_1$—$C_4$ hydrocarbons obtained can be seen in Table 17, Example 31A. After several hours, the catalyst was removed from the reactor, regenerated under air at 550°C for 16 hours then re-tested for methanol conversion. The conditions used and distribution of the $C_1$—$C_4$ hydrocarbons obtained can be seen in Table 17, Example 31B.

TABLE 17

| | Ex. 31A | Ex. 31B |
|---|---|---|
| Temp/°C | 400 | 400 |
| Conc of methanol in helium | 20% | 18% |
| WHSV methanol | 1 | 1 |
| Conversion | 100% | 100% |
| Distribution % (w/w) $C_1$—$C_4$ hydrocarbons obtained | | |
| Methane | 2.9 | 1.7 |
| Ethane | — | — |
| Ethylene | 6.9 | 7.4 |
| Propane | 6.0 | 6.4 |
| Propylene | 44.6 | 42.7 |
| Butanes | 3.0 | 3.3 |
| Butylenes | 36.7 | 38.5 |

Example 32

A sample of sodium TEPA Nu-10 prepared as described below was calcined and ion-exchanged by a procedure similar to that described in Example 1. Chemical analysis of the resulting material indicated that it had the following molar composition ignoring hydrogen:

$$0.017Na_2O \,.\, Al_2O_3 \,.\, 82SiO_2$$

In a continuous flow reactor, about 1 g of the catalyst, particle size 710—1000 μ, was tested for methanol conversion. The catalyst was activated at 450°C under helium for 1 hour then methanol in helium was passed over the catalyst. The reaction conditions and analysis of the $C_1$—$C_4$ hydrocarbons obtained can be seen in Table 18, Example 32A. After examination, the catalyst was removed from the reactor, regenerated under air at 550°C for 16 hours then re-tested for methanol conversion. After activation at 450°C for 1 hour under helium, the temperature was decreased to 350°C then 55% methanol in helium was passed over the catalyst. Reaction details and analysis of the $C_1$—$C_4$ hydrocarbons produced can be seen in Table 18, Example 32B.

TABLE 18

| | Ex. 32A | Ex. 32B |
|---|---|---|
| Temp/°C | 450 | 350 |
| Conc of methanol in helium | 65% | 55% |
| WHSV methanol | 1 | 1 |
| Conversion | 100% | >90% |
| Distribution % (w/w) $C_1$—$C_4$ hydrocarbons obtained. | | |
| Methane | 13.4 | 9.5 |
| Ethane | 1.7 | 1.4 |
| Ethylene | 11.3 | 3.5 |
| Propane | 13.0 | 20.3 |
| Propylene | 34.6 | 28.9 |
| Butanes | 1.3 | 6.1 |
| Butylenes | 24.8 | 30.4 |

For the preparation of the zeolite Nu-10, the reaction mixture had the following composition:

2.3 $Na_2O$, 27.5 TEPA, 94$SiO_2$, 3800$H_2O$, 53 NaCl

58 g tetraethylenepentamine were dispersed in 215 g Syton X-30 of molar composition 31.65 $Na_2O$, $Al_2O_3$, 2409$SiO_2$, 21076$H_2O$, followed by a solution of 2.1 g sodium aluminate (1.22 $Na_2O$, $Al_2O_3$, 1.2 $H_2O$) in 10 g water. Finally, a solution of 34.5 g sodium chloride in 598 g water was stirred in. Stirring continued for 30 minutes to homogenize the mixture. The reaction was carried out in a 1 litre stirred stainless steel autoclave for 114 hours at 150°C. After filtration, washing and drying, the product was sodium TEPA—Nu-10 with no detectable impurities other than a slight trace of sodium chloride.

Example 33

A sample of Nu-10 prepared and activated as in Example 32 was further tested for aromatics production from methanol. A mixture of methanol in nitrogen (65% methanol) was passed over the catalyst at 450°C. the reaction conditions and hydrocarbon analysis are given in Table 19.

TABLE 19

| | | |
|---|---|---|
| Temperature/°C | 450 | |
| Conc of methanol % in nitrogen | 65 | |
| WHSV methanol | 1 | |
| Methanol conversion % | 100 | |
| The hydrocarbons produced comprised: | $C_1$—$C_5$ aliphatics | 92 wt.% |
| | Aromatics | 7 wt.% |
| | Higher aliphatics | traces |
| Analysis of the aromatics showed: | Benzene | 1.5% |
| | Toluene | 16% |
| | Ethylbenzene | 3.5% |
| | Xylenes | 40% |
| | $C_9$ aromatics | 39% |

Example 34

The reaction mixture had the following molar composition:

$$5.9\ K_2O,\ 10\ TEPA,\ Al_2O_3,\ 30\ SiO_2,\ 1500\ H_2O$$

45 g Aerosil 200 were dispersed in a mixture of 47.3 g TEPA and 650 g water. Next, 3.9 g alumina were dissolved in 16.6 KOH and 27 g water. The aluminate solution was stirred into the slurry and the mixture was reacted for 64 hours at 180°C. The product was potassium TEPA Nu-10 containing traces α-cristobalite and a ZSM5 type zeolite. The $SiO_2/Al_2O_3$ ratio of this product was 25.

**Claims**

1. A synthetic zeolite material having a molar composition expressed by the formula:

$$0.5\ \text{to}\ 1.5\ R_2O{:}Y_2O_3{:}\ \text{at least}\ 20\ XO_2{:}0\ \text{to}\ 4000\ H_2O$$

wherein R is a monovalent cation or 1/n of a cation of valency n, X is silicon and/or germanium, Y is one or more of aluminium, iron, chromium, vanadium, molybdenum, arsenic, antimony, manganese, gallium or boron, and $H_2O$ is water of hydration additional to water notionally present when R is H, and having an X-ray pattern substantially as set out in the following Table:

| d(A) | I |
|---|---|
| 10.95±0.25 | m→s |
| 8.80±0.14 | w→m |
| 6.99±0.14 | w→m |
| 5.41±0.10 | w |
| 4.57±0.09 | w |
| 4.38±0.08 | vs |
| 3.69±0.07 | vs |
| 3.63±0.07 | vs |
| 3.48±0.06 | m→s |
| 3.36±0.06 | w |
| 3.31±0.05 | w |
| 2.78±0.05 | w |
| 2.53±0.04 | m |
| 2.44±0.04 | w |
| 2.37±0.03 | w |
| 1.88±0.02 | w |

vs=60 to 100
s=40 to 60
m=20 to 40
w= 0 to 20

2. A synthetic zeolite material according to claim 1 having a molar composition expressed by the formula:

$$0.5 \text{ to } 1.5\ H_2O:Y_2O_3: \text{ at least } 60\ XO_2:0 \text{ to } 200\ H_2O$$

3. A synthetic zeolite material according to claim 1 having a molar composition expressed by the formula:

$$0.5 \text{ to } 1.5\ H_2O:Y_2O_3:20 \text{ to } 5000\ XO_2:0 \text{ to } 4000\ H_2O$$

4. A synthetic zeolite material according to any one of claims 1 to 3 wherein R is or includes hydrogen.

5. A method of making a synthetic zeolite material as defined in claim 1 which comprises reacting an aqueous mixture containing at least one oxide $XO_2$, at least one oxide $Y_2O_3$ and at least one polyalkylene polyamine having the formula

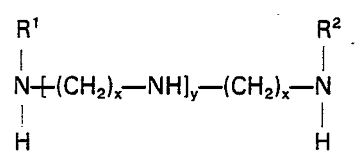

wherein each of $R^1$ and $R^2$, independently, represent hydrogen or a $C_1$—$C_6$ alkyl group, x is an integer from 2 to 6 and y is an integer from 0 to 10, provided that when y is 0, x is from 2 to 5 and wherein the aqueous mixture has the molar composition:

| | |
|---|---|
| $XO_2/Y_2O_3$ | 25 to 5000 |
| $M^1OH/XO_2$ | $10^{-8}$ to 1.0 |
| $H_2O/XO_2$ | 10 to 200 |
| $Q/XO_2$ | 0.05 to 4 |
| $M^2Z/XO_2$ | 0 to 4 |

wherein X and Y have the meanings given in claim 1, each of $M^1$ and $M^2$, independently, is an alkali metal, ammonium or hydrogen, Q is the polyalkylene polyamine and Z is a strong acid radical.

6. A method according to claim 5 wherein $XO_2/Y_2O_3$ is in the range 40 to 1000.

7. A method according to claim 6 wherein $XO_2/Y_2O_3$ is in the range 80 to 500.

8. A method according to any one of claims 5 to 7 wherein the polyalkylene polyamine is triethylene tetramine or tetraethylene pentamine.

9. A catalyst comprising a synthetic zeolite material as claimed in any one of claims 1 to 4.

10. A catalytic process employing the catalyst claimed in claim 9.

11. A process according to claim 10 wherein an alkylbenzene or a mixture of alkylbenzenes is reacted with an alkylating agent under alkylation conditions.

12. A process according to claim 11 wherein toluene is reacted with methanol to form a mixture of xylene isomers.

13. A process according to claim 10 wherein a lower monohydric alcohol or an ether derived therefrom is converted to hydrocarbons.

14. A process according to claim 13 wherein methanol or dimethyl ether is converted to lower olefins.

**Patentansprüche**

1. Synthetisches Zeolithmaterial mit einer molaren Zusammensetzung, die durch die Formel:

$$0{,}5 \text{ bis } 1{,}5\ R_2O{:}Y_2O_3{:}\ \text{mindestens } 20\ XO_2{:}0 \text{ bis } 4000\ H_2O$$

ausgedrückt wird, worin R ein einwertiges Kation oder 1/n eines Kations mit der Wertigkeit n ist, X Silicium und/oder Germanium ist, Y ein oder mehr als ein Element der Gruppe Aluminium, Eisen, Chrom, Vanadium, Molybdän, Arsen, Antimon, Mangan, Gallium und Bor ist und $H_2O$ Hydratwasser ist, das zu Wasser hinzukommt, das begrifflich vorhanden ist, wenn R H ist, und im wesentlichen mit einem Röntgenbeugungsbild, wie es in der folgenden Tabelle dargelegt ist:

| d (Å) | I |
|---|---|
| 10,95±0,25 | m→s |
| 8,80±0,14 | w→m |
| 6,99±0,14 | w→m |
| 5,41±0,10 | w |
| 4,57±0,09 | w |
| 4,38±0,08 | vs |
| 3,69±0,07 | vs |
| 3,63±0,07 | vs |
| 3,48±0,06 | m→s |
| 3,36±0,06 | w |
| 3,31±0,05 | w |
| 2,78±0,05 | w |
| 2,53±0,04 | m |
| 2,44±0,04 | w |
| 2,37±0,03 | w |
| 1,88±0,02 | w |

vs=60 bis 100
s=40 bis 60
m=20 bis 40
w= 0 bis 20

2. Synthetisches Zeolithmaterial nach Anspruch 1 mit einer molaren Zusammensetzung, die durch die Formel:

$$0{,}5 \text{ bis } 1{,}5\ H_2O{:}Y_2O_3{:}\ \text{mindestens } 60\ XO_2{:}0 \text{ bis } 200\ H_2O$$

ausgedrückt wird.

3. Synthetisches Zeolithmaterial nach Anspruch 1 mit einer molaren Zusammensetzung, die durch die Formel:

$$0{,}5 \text{ bis } 1{,}5\ H_2O{:}Y_2O_3{:}20 \text{ bis } 5000\ XO_2{:}0 \text{ bis } 4000\ H_2O$$

ausgedrückt wird.

4. Synthetisches Zeolithmaterial nach einem der Ansprüche 1 bis 3, worin R Wasserstoff ist oder Wasserstoff enthält.

5. Verfahren zur Herstellung eines synthetisches Zeolithmaterials, wie es in Anspruch 1 definiert ist, bei dem eine wäßrige Mischung, die mindestens ein Oxid $XO_2$, mindestens ein Oxid $Y_2O_3$ und mindestens ein Polyalkylenpolyamin mit der Formel:

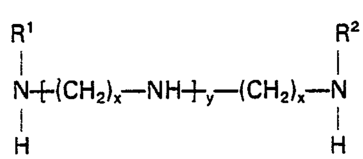

worin $R^1$ und $R^2$ unabhängig voneinander jeweils Wasserstoff oder eine $C_1$- bis $C_6$-Alkylgruppe bedeuten, x

eine ganze Zahl von 2 bis 6 ist und y eine ganze Zahl von 0 bis 10 ist, wobei die Bedingung gilt, daßx2 bis 5 ist, wenn y 0 ist, enthält, zur Reaktion gebracht wird, wobei die wäßrige Mischung die folgende molare Zusammensetzung hat:

| | |
|---|---|
| $XO_2/Y_2O_3$ | 25 bis 5000 |
| $M^1OH/XO_2$ | $10^{-8}$ bis 1,0 |
| $H_2O/XO_2$ | 10 bis 200 |
| $Q/XO_2$ | 0,05 bis 4 |
| $M^2Z/XO_2$ | 0 bis 4 |

worin X und Y die in Anspruch 1 angegebenen Bedeutungen haben, $M^1$ und $M^2$ unabhängig voneinander jeweils ein Alkalimetall, Ammonium oder Wasserstoff bedeuten, Q das Polyalkylenpolyamin ist und Z ein Säurerest einer starken Säure ist.

6. Verfahren nach Anspruch 5, bei dem $XO_2/Y_2O_3$ 40 bis 1000 beträgt.

7. Verfahren nach Anspruch 6, bei dem $XO_2/Y_2O_3$ 80 bis 500 beträgt.

8. Verfahren nach einem der Ansprüche 5 bis 7, bei dem das Polyalkylenpolyamin, Triethylentetramin oder Tetraethylenpentamin ist.

9. Katalysator, der aus einem synthetischen Zeolithmaterial nach einem der Ansprüche 1 bis 4 besteht.

10. Katalytisches Verfahren, bei dem der Katalysator nach Anspruch 9 verwendet wird.

11. Verfahren nach Anspruch 10, bei dem ein Alkylbenzol oder eine Mischung von Alkylbenzolen unter Alkylierungsbedingungen mit einem Alkylierungsmittel umgesetzt wird.

12. Verfahren nach Anspruch 11, bei dem Toluol mit Methanol umgesetzt wird, um eine Mischung von Xylolisomeren zu bilden.

13. Verfahren nach Anspruch 10, bei dem ein niederer einwertiger Alkohol oder ein davon abgeleiteter Ether in Kohlenwasserstoffe umgewandelt wird.

14. Verfahren nach Anspruch 13, bei dem Methanol oder Dimethylether in niedere Olefine umgewandelt wird.

**Revendications**

1. Zéolite de synthèse ayant une composition molaire exprimée par la formule ci-après:

$$0,5 \text{ à } 1,5\ R_2O:Y_2O_3: \text{au moins } 20\ XO_2:0 \text{ à } 4000\ H_2O$$

dans laquelle R est un cation monovalent ou 1/n d'un cation de valence n, X est du silicium et/ou du germanium, Y est un ou plusieurs des éléments aluminium, fer, chrome, vanadium, molybdène, arsenic, antimoine, manganèse, gallium ou bore, et $H_2O$ est de l'eau d'hydratation s'ajoutant à l'eau présente en théorie quand R est H, et ayant un spectre aux rayons X sensiblement tel qu'indiqué dans le Tableau suivant:

| d(A) | I |
|---|---|
| 10,95±0,25 | m→s |
| 8,80±0,14 | w→m |
| 6,99±0,14 | w→m |
| 5,41±0,10 | w |
| 4,57±0,09 | w |
| 4,38±0,08 | vs |
| 3,69±0,07 | vs |
| 3,63±0,07 | vs |
| 3,48±0,06 | m→s |
| 3,36±0,06 | w |
| 3,31±0,05 | w |
| 2,78±0,05 | w |
| 2,53±0,04 | m |
| 2,44±0,04 | w |
| 2,37±0,03 | w |
| 1,88±0,02 | w |

vs=60 à 100
s=40 à 60
m=20 à 40
w= 0 à 20

2. Zéolite synthétique suivant la revendication 1, ayant une composition molare exprimée par la formule:

$$0,5 \text{ à } 1,5\ H_2O:Y_2O_3: \text{ au moins } 60\ XO_2:0 \text{ à } 200\ H_2O$$

3. Zéolite synthétique suivant la revendication 1, ayant une composition molaire exprimée par la formule:

$$0,5 \text{ à } 1,5\ H_2O:Y_2O_3:20 \text{ à } 5000\ XO_2:0 \text{ à } 4000\ H_2O$$

4. Zéolite synthétique suivant l'une quelconque des revendications 1 à 3, dans laquelle R est ou comprend de l'hydrogène.

5. Procédé de fabrication d'une zéolite synthétique telle que définie dans la revendication 1, selon lequel on fait réagir un mélange aqueux contenant au moins un oxyde $XO_2$, au moins un oxyde $Y_2O_3$ et au moins une polyalcoylène-polyamine représentée par la formule

$$\begin{array}{c} R^1 \\ | \\ N \\ | \\ H \end{array}\!\!+\!(CH_2)_x\!-\!NH\!+_y\!-\!(CH_2)_x\!-\!\begin{array}{c} R^2 \\ | \\ N \\ | \\ H \end{array}$$

dans laquelle chacun des radicaux $R^1$ et $R^2$ représente indépendamment, de l'hydrogène ou un groupe alcoyle en $C_1$—$C_6$, x est un nombre entier de 2 à 6 et y est un nombre entier de 0 à 10, à condition que lorsque y est 0, x ait une valeur de 2 à 5, suivant lequel le mélange aqueux a la composition molaire:

| | |
|---|---|
| $XO_2/Y_2O_3$ | 25 à 5000 |
| $M^1OH/XO_2$ | $10^{-8}$ à 1,0 |
| $H_2O/XO_2$ | 10 à 200 |
| $Q/XO_2$ | 0,05 à 4 |
| $M^2Z/XO_2$ | 0 à 4 |

où X et Y ont les significations indiquées dans la revendication 1, $M^1$ et $M^2$ sont chacun, indépendamment, un métal alcalin, de l'ammonium ou de l'hydrogène, Q est la polyalcoylène-polyamine et Z est un radical d'acide fort.

6. Procédé suivant la revendication 5, caractérisé en ce que le rapport $XO_2/Y_2O_3$ est compris entre 40 et 1000.

7. Procédé suivant la revendication 6, caractérisé en ce que le rapport $XO_2/Y_2O_3$ est compris entre 80 et 500.

8. Procédé suivant l'une quelconque des revendications 5 à 7, caractérisé en ce que la polyalcoylène-polyamine est la triéthylènetétramine ou la tétraéthylène pentamine.

9. Catalyseur renfermant une zéolite synthétique suivant l'une quelconque des revendications 1 à 4.

10. Procédé catalytique utilisant un catalyseur tel que revendiqué dans la revendication 9.

11. Procédé suivant la revendication 10, caractérisé en ce qu'on fait réagir un alcoylbenzène ou un mélange d'alcoylbenzènes avec un agent d'alcoylation dans des conditions d'alcoylation.

12. Procédé suivant la revendication 11, caractérisé en ce qu'on fait réagir du toluène avec du méthanol pour former un mélange d'isomères de xylène.

13. Procédé suivant la revendication 10, caractérisé en ce qu'on convertit un alcool monohydrique inférieur ou un éther dérivé de celui-ci en hydrocarbures.

14. Procédé suivant la revendication 13, caractérisé en ce qu'on convertit du méthanol ou de l'éther diméthylique en oléfines inférieures.

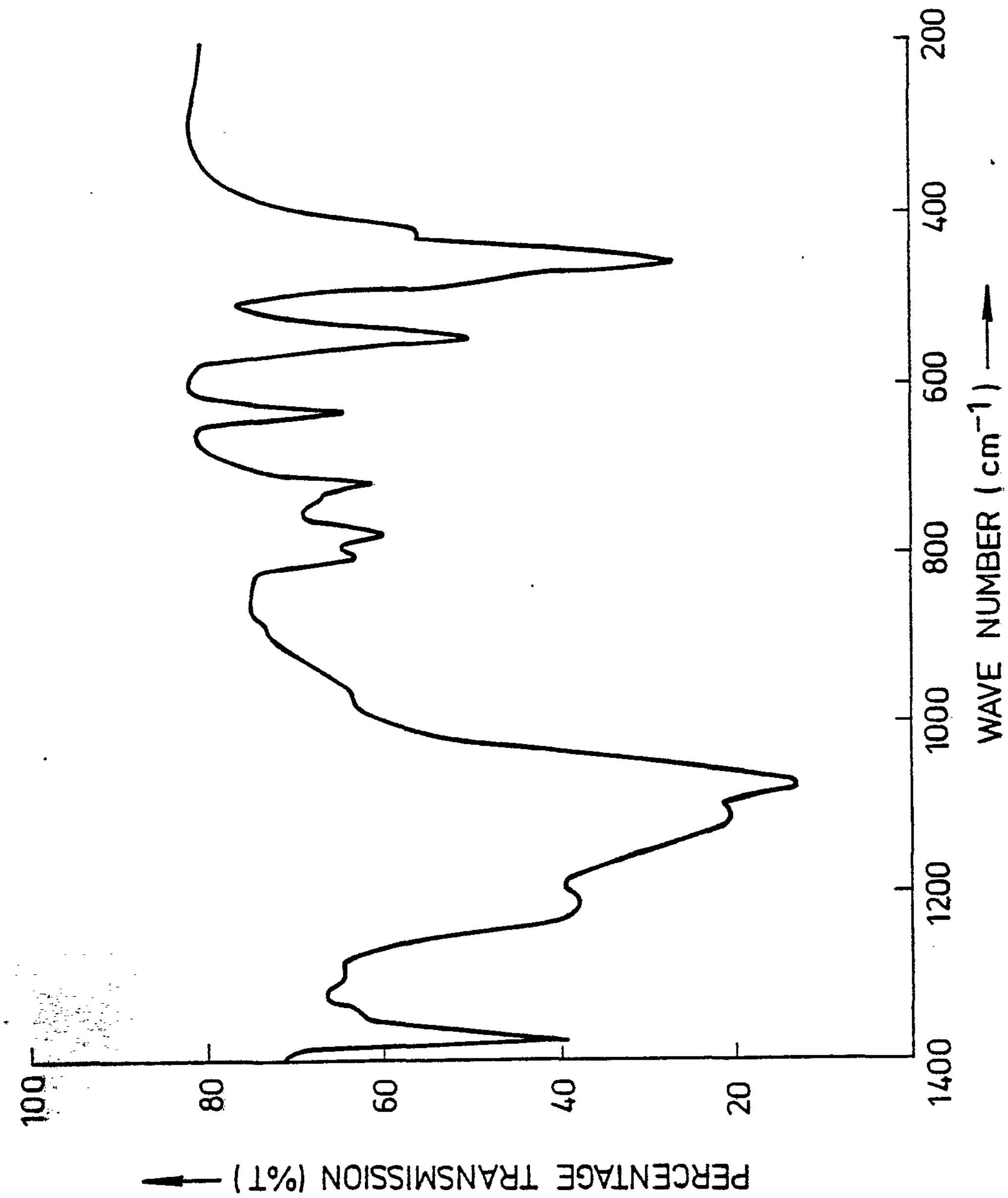
PERCENTAGE TRANSMISSION (%T)
100
80
60
40
20
WAVE NUMBER (cm-1)
1400
1200
1000
800
600
400
200